# EUROPÄISCHE PATENTSCHRIFT

(11) **EP 2 379 071 B1**
(45) Veröffentlichungstag und Bekanntmachung des Hinweises auf die Patenterteilung: **08.05.2013**
(21) Anmeldenummer: 09768334.6
(22) Anmeldetag: 08.12.2009
(51) Int. Cl.: C07D 471/04, A61K 31/33, A61P 9/00

(54) **NEUE ALIPHATISCH SUBSTITUIERTE PYRAZOLOPYRIDINE UND IHRE VERWENDUNG**
NOVEL ALIPHATICALLY SUBSTITUTED PYRAZOLOPYRIDINES, AND THE USE THEREOF
NOUVELLES PYRAZOLOPYRIDINES À SUBSTITUTION ALIPHATIQUE ET LEUR UTILISATION

(30) Priorität: 19.12.2008 DE 102008063992
(43) Veröffentlichungstag der Anmeldung: 26.10.2011
(73) Patentinhaber: Bayer Intellectual Property GmbH, 40789 Monheim (DE)
(72) Erfinder: SCHIROK, Hartmut, 40764 Langenfeld (DE); GRIEBENOW, Nils, 41541 Dormagen (DE); FÜRSTNER, Chantal, 45478 Mülheim/Ruhr (DE); MITTENDORF, Joachim, 42113 Wuppertal (DE); STASCH, Johannes-Peter, 42651 Solingen (DE); WUNDER, Frank, 42117 Wuppertal (DE); HEITMEIER, Stefan, 42489 Wülfrath (DE)
(74) Vertreter: BIP Patents
(86) Internationale Anmeldenummer: PCT/EP2009/008741
(87) Internationale Veröffentlichungsnummer: WO 2010/078900

(56) Entgegenhaltungen:
- WO-A1-2007/128454

## Beschreibung

Die vorliegende Anmeldung betrifft neue aliphatisch substituierte Pyrazolopyridine, Verfahren zu ihrer Herstellung sowie ihre Verwendung zur Herstellung von Arzneimitteln zur Behandlung und/oder Prävention von Krankheiten, insbesondere zur Behandlung und/oder Prävention von Herz-Kreislauf-Erkrankungen.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriphosphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch Kohlenmonoxid (CO) ist in der Lage, an das Eisen-Zentralatom des Häms zu binden, wobei die Stimulierung durch CO deutlich geringer ist als die durch NO.

Durch die Bildung von cGMP und der daraus resultierenden Regulation von Phosphodiesterasen, Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion, der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Atherosklerose, Angina pectoris, Herzinsuffizienz, Myokardinfarkt, Thrombosen, Schlaganfall und sexueller Dysfunktion führen kann.

Eine auf die Beeinflussung des cGMP-Signalweges in Organismen abzielende NO-unabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch Biokonversion gebildet und aktiviert die lösliche Guanylatcyclase durch Angriff am Eisen-Zentralatom des Häms. Neben den Nebenwirkungen gehört die Toleranzentwicklung zu den entscheidenden Nachteilen dieser Behandlungsweise.

In den letzten Jahren wurden einige Substanzen beschrieben, die die lösliche Guanylatcyclase direkt, d.h. ohne vorherige Freisetzung von NO stimulieren, wie beispielsweise 3-(5'-Hydroxymethyl-2'-furyl)-1-benzylindazol [YC-1; Wu et al., Blood 84 (1994), 4226; Mülsch et al., Brit. J. Pharmacol. 120 (1997), 681], Fettsäuren [Goldberg et al., J. Biol. Chem. 252 (1977), 1279], Diphenyliodonium-hexafluorophosphat [Pettibone et al., Eur. J. Pharmacol. 116 (1985), 307], Isoliquiritigenin [Yu et al., Brit. J. Pharmacol. 114 (1995), 1587] sowie verschiedene substituierte Pyrazol-Derivate (WO 98/16223).

Anellierte Pyrazol-Derivate sind unter anderem in WO 98/23619, WO 00/06568, WO 00/06569, WO 02/42299, WO 02/42300, WO 02/42301, WO 02/42302, WO 02/092596, WO 03/004503, WO 03/095451, WO 2007/124854, WO 2007/128454 und WO 2008/031513 als Stimulatoren der löslichen Guanylatcyclase beschrieben. Allerdings zeigte es sich, dass diese Verbindungen zum Teil bezüglich ihrer physikochemischen Eigenschaften, wie beispielsweise ihrer Löslichkeit, oder hinsichtlich ihrer in *vivo*-Eigenschaften, wie beispielsweise ihrem Verhalten in der Leber, ihrem pharmakokinetischen Verhalten, ihrer Dosis-Wirkungsbeziehung und/oder ihrem Metabolisierungsweg, Nachteile aufweisen.

Weiterhin werden WO 01/57024, WO 03/035005 sowie WO 2005/030121 verschiedene anellierte Pyrazol-Derivate zur Behandlung von Erkrankungen offenbart.

Aufgabe der vorliegenden Erfindung war die Bereitstellung neuer Substanzen, die als Stimulatoren der löslichen Guanylatcyclase wirken und ein gleiches oder verbessertes physikochemisches, pharmakokinetisches und/ oder therapeutisches Profil gegenüber den aus dem Stand der Technik bekannten Verbindungen aufweisen.

Gegenstand der vorliegenden Erfindung sind Verbindungen der allgemeinen Formel (I) 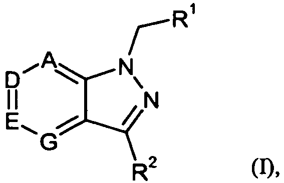 in welcher
A für CR³ oder N steht,
   wobei
   R³ für Wasserstoff, Halogen, Cyano, (C₁-C₄)-Alkyl, Trifluormethyl, Amino, (C₁-C₄)-Alkoxy und Trifluormethoxy steht,
D für CR⁴ oder N steht,
   wobei
   R⁴ für Wasserstoff, Halogen, Cyano, (C₁-C₄)-Alkyl, Trifluormethyl, Amino, (C₁-C₄)-Alkoxy und Trifluormethoxy steht,
E für CR⁵ oder N steht,
   wobei
   R⁵ für Wasserstoff, Halogen, Cyano, (C₁-C₄)-Alkyl, Trifluormethyl, Amino, (C₁-C₄)-Alkoxy und Trifluormethoxy steht,
G für CR⁶ oder N steht,
   wobei
   R⁶ für Wasserstoff, Halogen, Cyano, (C₁-C₄)-Alkyl, Trifluormethyl, Amino, (C₁-C₄)-Alkoxy und Trifluormethoxy steht,
   mit der Maßgabe, dass maximal 2 der Gruppen A, D, E und G für N stehen,
R¹ für (C₃-C₈)-Cycloalkyl steht,
   wobei (C₃-C₈)-Cycloalkyl mit 1 oder 2 Substituenten unabhängig voneinander ausgewählt aus der Gruppe Fluor und (C₁-C₄)-Alkyl substituiert sein kann,
R² für Pyrrolinyl, Dihydropyrazolyl, Imidazolinyl, Dihydrooxazolyl, Dihydroisoxazolyl, Dlhydro-1,2,4-triazolyl, Dlhydro-1,2,4-oxadiazolyl, Dihydro-1,3,4-oxadiazolyl, Dihydro-1,2,4-thiadiazolyl, Dihydropyranyl, 1,4-Dihydropyridyl, Tetrahydropyrimidinyl, 1,3-Oxazinyl, Furyl, Thienyl, Thiazolyl, Oxazolyl, Isothiazolyl, Isoxazolyl, Pyrazolyl, Imidazolyl, Triazolyl, Oxadiazolyl, Thiadiazolyl, Pyridyl, Pyrimidinyl, Pyridazinyl, Pyrazinyl oder Triazinyl steht, wobei Pyrrolinyl, Dihydropyrazolyl, Imidazolinyl, Dihydrooxazolyl, Dihydroisoxazolyl, Dihydro-1,2,4-triazolyl, Dihydro-1,2,4-oxadiazolyl, Dihydro-1,3,4-oxadiazolyl, Dihydro-1,2,4-thiadiazolyl, Dihydropyranyl, 1,4-Dihydropyridyl, Tetrahydropyrimidinyl, 1,3-Oxazinyl, Furyl, Thienyl, Thiazolyl, Oxazolyl, Isothiazolyl, Isoxazolyl, Pyrazolyl, Imidazolyl, Triazolyl, Oxadiazolyl, Thiadiazolyl, Pyridyl, Pyrimidinyl, Pyridazinyl, Pyrazinyl und Triazinyl mit 1 bis 4 Substituenten unabhängig voneinander ausgewählt aus der Gruppe Halogen, Azido, Nitro, Cyano, -R⁷, -C(=O)-R⁷, -C(=O)-OR⁷, -C(=O)-NR⁷R⁸, -O-(C=O)ₙ-R⁷, -O-C(=O)-OR⁷, -O-C(=O)-NR⁷R⁸, -S(O)ₚ-R⁷, -SO₂-OR⁷, -SO₂-NR⁷R⁸, -NR⁷-(C=O)ₙ-R⁸, -NR⁷-SO₂-R⁸, -NR⁷-C(=O)-OR⁸, -NR⁹-C(=O)-NR⁷R⁸ und -NR⁹-SO₂-NR⁷R⁸ substituiert sein kann,
   worin
   n für eine Zahl 0 oder 1 steht,
   p für eine Zahl 0, 1 oder 2 steht,
   R⁷, R⁸ und R⁹ jeweils unabhängig voneinander für Wasserstoff, (C₁-C₆)-Alkyl, (C₂-C₆)-Alkenyl, (C₃-C₈)-Cycloalkyl, (C₃-C₈)-Cycloalkenyl, (C₆-C₁₀)-Aryl, 4- bis 8-gliedriges Heterocyclyl oder 5- bis 10-gliedriges Heteroaryl stehen,
   worin R⁷, R⁸ und R⁹ ihrerseits mit 1 bis 5 Substituenten unabhängig voneinander ausgewählt aus der Gruppe Halogen, Azido, Nitro, Cyano, Trifluormethyl, (C₁-C₆)-Alkyl, (C₁-C₆)-Alkylcarbonyl, (C₁-C₆)-Alkylcarbonyloxy, Hydroxycarbonyl, (C₁-C₆)-Alkoxycarbonyl, Aminocarbonyl, Mono-(C₁-C₆)-alkylaminocarbonyl, Di-(C₁-C₆)-alkylaminocarbonyl, Hydroxy, Trifluormethoxy, (C₁-C₆)-Alkoxy, Oxo, Mercapto, (C₁-C₆)-Alkylthio, Amino, Mono-(C₁-C₆)-alkylamino, Di-(C₁-C₆)-alkylamino, Formylamino, (C₁-C₆)-Alkylcarbonylamino, (C₁-C₆)-Alkoxycarbonylamino, (C₃-C₈)-Cycloalkyl, (C₃-C₈)-Cycloalkenyl sowie 4- bis 8-gliedriges Heterocyclyl substituiert sein können,
   oder
   R⁷ und R⁸ zusammen mit dem Rest, an den sie jeweils beide gebunden sind, einen 4- bis 8-gliedrigen Heterocyclus bilden,
   oder
   R⁷ und R⁹ zusammen mit dem Rest, an den sie jeweils beide gebunden sind, einen 4- bis 8-gliedrigen Heterocyclus bilden,
sowie ihre *N*-Oxide, Salze, Solvate, Salze der *N*-Oxide und Solvate der *N*-Oxide und Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Erfindungsgemäße Verbindungen sind ebenso *N*-Oxide der Verbindungen der Formel (I) sowie deren Salze, Solvate und Solvate der Salze.

Die erfindungsgemäßen Verbindungen können in Abhängigkeit von ihrer Struktur in stereoisomeren Formen (Enantiomere, Diastereomere) existieren. Die vorliegende Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Trisethanolamin, Dicyclohexylamin, Dimethylaminoethanol, Prokain, Dibenzylamin, *N*-Methylmorpholin, Arginin, Lysin, Ethylendiamin und *N*-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Im Rahmen der vorliegenden Erfindung haben die Substituenten, soweit nicht anders spezifiziert, die folgende Bedeutung:
Alkyl steht im Rahmen der Erfindung für einen linearen oder verzweigten Alkylrest mit der jeweils angegebenen Anzahl an Kohlenstoffatomen. Beispielhaft und vorzugsweise seien genannt: Methyl, Ethyl, n-Propyl, Isopropyl, n-Butyl, iso-Butyl, 1-Methylpropyl, tert.-Butyl, n-Pentyl, iso-Pentyl, 1-Ethylpropyl, 1-Methylbutyl, 2-Methylbutyl, 3-Methylbutyl, n-Hexyl, 1-Methylpentyl, 2-Methylpentyl, 3-Methylpentyl, 4-Methylpentyl, 3,3-Dimethylbutyl, 1-Ethylbutyl und 2-Ethylbutyl.
Cycloalkyl steht in Rahmen der Erfindung für einen monocyclischen, gesättigten Alkylrest mit 3 bis 8 Kohlenstoffatomen. Beispielhaft und vorzugsweise seien genannt: Cyclopropyl, Cyclobutyl, Cyclopentyl, Cyclohexyl, Cycloheptyl und Cyclooctyl.
Alkenyl steht im Rahmen der Erfindung für einen linearen oder verzweigten Alkenylrest mit 2 bis 6 Kohlenstoffatomen und einer oder zwei Doppelbindungen. Bevorzugt ist ein geradkettiger oder verzweigter Alkenylrest mit 2 bis 4 Kohlenstoffatomen und einer Doppelbindung. Beispielhaft und vorzugsweise seien genannt: Vinyl, Allyl, Isopropenyl und n-But-2-en-1-yl.
Cycloalkenyl steht in Rahmen der Erfindung für einen monocyclischen Carbocyclus mit 3 bis 8 Kohlenstoffatomen und einer Doppelbindung. Beispielhaft und vorzugsweise seien genannt: Cyclobutenyl, Cyclopentenyl, Cyclohexenyl, Cycloheptenyl und Cyclooctenyl.
Alkylcarbonyl steht im Rahmen der Erfindung für einen linearen oder verzweigten Alkylrest mit 1 bis 6 bzw. 1 bis 4 Kohlenstoffatomen in der Alkylkette und einer in 1-Position angebundenen Carbonylgruppe. Beispielhaft und vorzugsweise seien genannt: Methylcarbonyl, Ethylcarbonyl, n-Propylcarbonyl, iso-Propylcarbonyl, n-Butylcarbonyl, iso-Butylcarbonyl und tert.-Butylcarbonyl.
Alkylcarbonyloxy steht im Rahmen der Erfindung für einen linearen oder verzweigten Alkylcarbonylrest, der über ein Suaerstoffatom gebunden ist und 1 bis 6 bzw. 1 bis 4 Kohlenstoffatomen in der Alkylkette trägt. Beispielhaft und vorzugsweise seien genannt: Methylcarbonyloxy, Ethylcarbonyloxy, n-Propylcarbonyloxy, iso-Propylcarbonyloxy, n-Butylcarbonyloxy, iso-Butylcarbonyloxy und tert.-Butylcarbonyloxy.
Alkoxy steht im Rahmen der Erfindung für einen linearen oder verzweigten Alkoxyrest mit 1 bis 4 Kohlenstoffatomen. Beispielhaft und vorzugsweise seien genannt: Methoxy, Ethoxy, n-Propoxy, Isopropoxy, 1-Methylpropoxy, n-Butoxy, iso-Butoxy und tert.-Butoxy.
Alkylthio steht im Rahmen der Erfindung für einen linearen oder verzweigten Alkylthiorest mit 1 bis 6 bzw. 1 bis 4 Kohlenstoffatomen. Beispielhaft und vorzugsweise seien genannt: Methylthio, Ethylthio, n-Propylthio, Isopropylthio, n-Butylthio, tert.-Butylthio, n-Pentylthio und n-Hexylthio.
Alkoxycarbonyl stehen im Rahmen der Erfindung für einen linearen oder verzweigten Alkoxyrest mit 1 bis 6 bzw. 1 bis 4 Kohlenstoffatomen und einer am Sauerstoff angebundenen Carbonylgruppe. Bevorzugt ist ein linearer oder verzweigter Alkoxycarbonylrest mit 1 bis 4 Kohlenstoffatomen in der Alkoxy-Gruppe. Beispielhaft und vorzugsweise seien genannt: Methoxycarbonyl, Ethoxycarbonyl, n-Propoxycarbonyl, Isopropoxycarbonyl und tert.-Butoxycarbonyl.
Mono-alkylamino steht im Rahmen der Erfindung für eine Amino-Gruppe mit einem linearen oder verzweigten Alkylsubstituenten, der 1 bis 6 Kohlenstoffatome aufweist. Beispielhaft und vorzugsweise seien genannt: Methylamino, Ethylamino, n-Propylamino, Isopropylamino und tert.-Butylamino.
Di-alkylamino steht im Rahmen der Erfindung für eine Amino-Gruppe mit zwei gleichen oder verschiedenen linearen oder verzweigten Alkylsubstituenten, die jeweils 1 bis 6 Kohlenstoffatome aufweisen. Beispielhaft und vorzugsweise seien genannt: *N,N*-Dimethylamino, *N,N*-Diethylamino, *N*-Ethyl-*N-*methylamino, *N*-Methyl-*N*-n-propylamino, *N*-Isopropyl-*N*-n-propylamino, *N*-tert.-Butyl-*N*-methylamino, *N*-Ethyl-*N*-n-pentylamino und *N*-n-Hexyl-*N*-methylamino.
Mono-alkylaminocarbonyl steht im Rahmen der Erfindung für eine Amino-Gruppe, die über eine Carbonylgruppe verknüpft ist und die einen linearen oder verzweigten Alkylsubstituenten mit 1 bis 6 bzw. 1 bis 4 Kohlenstoffatomen aufweist. Bevorzugt ist ein Mono-alkylaminocarbonyl-Rest mit 1 bis 4 Kohlenstoffatomen in der Alkylgruppe. Beispielhaft und vorzugsweise seien genannt: Methylaminocarbonyl, Ethylaminocarbonyl, *n*-Propylaminocarbonyl, Isopropylaminocarbonyl, n-Butylaminocarbonyl, *tert*.-Butylaminocarbonyl, n-Pentylaminocarbonyl und n-Hexylaminocarbonyl.
Di-alkylaminocarbonyl steht im Rahmen der Erfindung für eine Amino-Gruppe, die über eine Carbonylgruppe verknüpft ist und die zwei gleiche oder verschiedene lineare oder verzweigte Alkylsubstituenten mit jeweils 1 bis 6 bzw. 1 bis 4 Kohlenstoffatomen aufweist. Bevorzugt ist ein Dialkylaminocarbonyl-Rest mit jeweils 1 bis 4 Kohlenstoffatomen pro Alkylgruppe. Beispielhaft und vorzugsweise seien genannt: *N,N-*Dimethylaminocarbonyl, *N,N-*Diethylaminocarbonyl, *N-*Ethyl-*N*-methylaminocarbonyl, *N*-Methyl-*N*-*n*-propylaminocarbonyl, *N*-*n*-Butyl-*N*-methylaminocarbonyl, *N*-tert.-Butyl-*N*-methylaminocarbonyl, *N-n*-Pentyl-*N*-methylaminocarbonyl und *N-n*-Hexyl-*N-*methylaminocarbonyl.
Alkylcarbonylamino steht im Rahmen der Erfindung für eine Amino-Gruppe mit einem linearen oder verzweigten Alkylcarbonyl-Substituenten, der 1 bis 6 bzw. 1 bis 4 Kohlenstoffatome in der Alkylkette aufweist und über die Carbonylgruppe mit dem N-Atom verknüpft ist. Beispielhaft und vorzugsweise seien genannt: Methylcarbonylamino, Ethylcarbonylamino, Propylcarbonylamino, n-Butylcarbonylamino, iso-Butylcarbonylamino und tert.-Butylcarbonylamino.
Alkoxycarbonylamino steht im Rahmen der Erfindung für eine Amino-Gruppe mit einem linearen oder verzweigten Alkoxycarbonyl-Substituenten, der 1 bis 6 bzw. 1 bis 4 Kohlenstoffatome in der Alkylkette aufweist und über die Carbonylgruppe mit dem N-Atom verknüpft ist. Beispielhaft und vorzugsweise seien genannt: Methoxycarbonylamino, Ethoxycarbonylamino, Propoxycarbonylamino, n-Butoxycarbonylamino, iso-Butoxycarbonylamino und tert.-Butoxycarbonylamino.
5- bis 10-gliedriges Heteroaryl steht im Rahmen der Erfindung für einen mono- oder gegebenenfalls bicyclischen aromatischen Heterocyclus (Heteroaromaten) mit insgesamt 5 bis 10 Ringatomen, der bis zu drei gleiche oder verschiedene Ring-Heteroatome aus der Reihe N, O und/oder S enthält und über ein Ring-Kohlenstoffatom oder gegebenenfalls über ein Ring-Stickstoffatom verknüpft ist. Beispielhaft seien genannt: Furyl, Pyrrolyl, Thienyl, Pyrazolyl, Imidazolyl, Thiazolyl, Oxazolyl, Isoxazolyl, Isothiazolyl, Triazolyl, Oxadiazolyl, Thiadiazolyl, Pyridyl, Pyrimidinyl, Pyridazinyl, Pyrazinyl, Triazinyl, Benzofuranyl, Benzothienyl, Benzimidazolyl, Benzoxazolyl, Benzothiazolyl, Benzotriazolyl, Indolyl, Indazolyl, Chinolinyl, Isochinolinyl, Naphthyridinyl, Chinazolinyl, Chinoxalinyl, Phthalazinyl, Pyrazolo[3,4-b]pyridinyl. Bevorzugt sind monocyclische 5- oder 6-gliedrige Heteroaryl-Reste mit bis zu drei Ring-Heteroatomen aus der Reihe N, O und/oder S wie beispielsweise Furyl, Thienyl, Thiazolyl, Oxazolyl, Isothiazolyl, Isoxazolyl, Pyrazolyl, Imidazolyl, Triazolyl, Oxadiazolyl, Thiadiazolyl, Pyridyl, Pyrimidinyl, Pyridazinyl, Pyrazinyl, Triazinyl.
Ein 4- bis 8-gliedriger Heterocyclus steht im Rahmen der Erfindung für einen monocyclischen, gesättigten Heterocyclus mit insgesamt 4 bis 8 Ringatomen, der ein oder zwei Ring-Heteroatome aus der Reihe N, O, S, SO und/oder SO₂ enthält und über ein Ring-Kohlenstoffatom oder gegebenenfalls ein Ring-Stickstoffatom verknüpft ist. Bevorzugt ist ein 5- bis 7-gliedriger Heterocyclus mit ein oder zwei Ring-Heteroatomen aus der Reihe N, O und/oder S, besonders bevorzugt ein 5-oder 6-gliedriger Heterocyclus mit ein oder zwei Ring-Heteroatomen aus der Reihe N und/oder O. Beispielhaft seien genannt: Azetidinyl, Oxetanyl, Pyrrolidinyl, Pyrazolidinyl, Tetrahydrofuranyl, Thiolanyl, Piperidinyl, Piperazinyl, Tetrahydropyranyl, Tetrahydrothiopyranyl, Morpholinyl, Thiomorpholinyl, Hexahydroazepinyl und Hexahydro-1,4-diazepinyl. Bevorzugt sind Pyrrolidinyl, Tetrahydrofuranyl, Piperidinyl, Piperazinyl, Tetrahydropyranyl und Morpholinyl.
Halogen schließt im Rahmen der Erfindung Fluor, Chlor, Brom und Iod ein. Bevorzugt sind Chlor oder Fluor.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt. Ganz besonders bevorzugt ist die Substitution mit einem Substituenten.

Bevorzugt im Rahmen der vorliegenden Erfindung sind Verbindungen der Formel (I), in welcher
A für CR³ oder N steht,
   wobei
   R³ für Wasserstoff steht,
D für CR⁴ oder N steht,
   wobei
   R⁴ für Wasserstoff steht,
E für CR⁵ oder N steht,
   wobei
   R⁵ für Wasserstoff steht,
G für CR⁶ oder N steht,
   wobei
   R⁶ für Wasserstoff steht,
   mit der Maßgabe, dass maximal 2 der Gruppen A, D, E und G für N stehen,
R¹ für Cyclopentyl, Cyclohexyl oder Cycloheptyl steht,
   wobei Cyclopentyl, Cyclohexyl oder Cycloheptyl mit 1 oder 2 Substituenten unabhängig voneinander ausgewählt aus der Gruppe Fluor und Methyl substituiert sein können,
R² für eine Gruppe der Formel 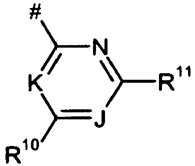 steht,
   wobei
   # für die Anknüpfstelle an den Heterobicyclus steht,
   K für CH oder N steht,
   J für CR¹², N oder N⁺-O⁻ steht,
   worin
   R¹² für Halogen, Nitro, Cyano, -R⁷, -C(=O)-R⁷, -C(=O)-OR⁷, -C(=O)-NR⁷R⁸, -O-(C=O)ₙ-R⁷, -O-C(=O)-OR⁷, -O-C(=O)-NR⁷R⁸, -S(O)ₚ-R⁷, -SO₂-OR⁷, -SO₂-NR⁷R⁸, -NR⁷-(C=O)ₙ-R⁸, -NR₇-SO₂-R⁸, -NR⁷-C(=O)-OR⁸, -NR⁹-C(=O)-NR⁷R⁸ und -NR⁹-SO₂-NR⁷R⁸ steht,
   worin
   n die Zahl 0 oder 1 darstellt,
   p die Zahl 0 oder 2 darstellt,
   R⁷, R⁸ und R⁹ jeweils unabhängig voneinander für Wasserstoff, (C₁-C₆)-Alkyl, (C₂-C₆)-Alkenyl, (C₃-C₇)-Cycloalkyl, (C₃-C₇)-Cycloalkenyl, Phenyl, 5- bis 7-gliedriges Heterocyclyl oder 5- oder 6-gliedriges Heteroaryl stehen,
   worin R⁷, R⁸ und R⁹ ihrerseits mit 1 bis 3 Substituenten unabhängig voneinander ausgewählt aus der Gruppe Fluor, Chlor, Cyano, Trifluormethyl, (C₁-C₄)-Alkyl, Hydroxy, Trifluormethoxy, (C₁-C₄)-Alkoxy, Oxo, Amino, Mono-(C₁-C₄)-alkylamino und Di-(C₁-C₄)-alkylamino substituiert sein können,
   oder
   R⁷ und R⁸ zusammen mit dem Rest, an den sie jeweils beide gebunden sind, einen 5- bis 7-gliedrigen Heterocyclus bilden,
   oder
   R⁷ und R⁹ zusammen mit dem Rest, an den sie jeweils beide gebunden sind, einen 5- bis 7-gliedrigen Heterocyclus bilden,
sowie ihre *N*-Oxide, Salze, Solvate, Salze der *N*-Oxide und Solvate der *N*-Oxide und Salze.

Besonders bevorzugt im Rahmen der vorliegenden Erfindung sind Verbindungen der Formel (I), in welcher
A für N steht,
D für CR⁴ steht,
   wobei
   R⁴ für Wasserstoff steht,
E für CR⁵ steht,
   wobei
   R⁵für Wasserstoff steht,
G für CR⁶ steht,
   wobei
   R⁶ für Wasserstoff steht,
R¹ für Cyclohexyl oder Cycloheptyl steht,
R² für eine Gruppe der Formel 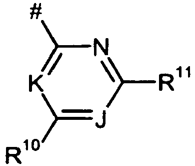 steht,
   wobei
   # für die Anknüpfstelle an den Heterobicyclus steht,
   K für N steht,
   J für CR¹² oder N steht,
   worin
   R¹² für Wasserstoff, -R⁷, -NR⁷-(C=O)ₙ-R⁸, -NR⁷-C(=O)-OR⁸ oder Pyridyl steht,
   worin
   n für die Zahl 1 steht,
   R⁷ für Wasserstoff, Trifluormethyl oder (C₁-C₄)-Alkyl steht,
   worin (C₁-C₄)-Alkyl seinerseits mit einem Substituenten ausgewählt aus der Gruppe Fluor, Trifluormethyl, Hydroxy und Methoxy substituiert sein kann,
   R⁸ für (C₁-C₄)-Alkyl oder (C₃-C₇)-Cycloalkyl steht, worin (C₁-C₄)-Alkyl und (C₃-C₇)-Cycloalkyl ihrerseits mit einem Substituenten ausgewählt aus der Gruppe Fluor, Trifluormethyl, (C₁-C₄)-Alkyl, Hydroxy, Methoxy und Oxo substituiert sein können,
   oder
   R⁷ und R⁸ zusammen mit dem Rest, an den sie jeweils beide gebunden sind, einen 5- bis 7-gliedrigen Heterocyclus bilden,
sowie ihre *N*-Oxide, Salze, Solvate, Salze der *N*-Oxide und Solvate der *N*-Oxide und Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Besonders bevorzugt sind Kombinationen von zwei oder mehreren der oben genannten Vorzugsbereiche.

Die erfindungsgemäßen Verbindungen der Formel (I) können hergestellt werden, indem man eine Verbindung der Formel (II) 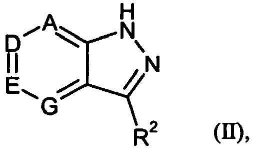 in welcher A, D, E, G und R² jeweils die oben angegebenen Bedingungen haben,
in einem inerten Lösungsmittel in Gegenwart einer geeigneten Base mit einer Verbindung der Formel (III) 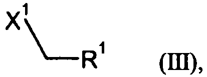 in welcher R¹ die oben angegebene Bedeutung hat
und
X¹ für eine geeignete Abgangsgruppe, wie Tosylat, Mesylat oder Halogen, insbesondere Brom, steht, umsetzt,
gegebenenfalls die resultierende Verbindung der Formel (I) nach literaturüblichen Verfahren weiter im oben angegebenen Bedeutungsumfang der einzelnen Substituenten und Reste modifiziert
und die so erhaltenen erfindungsgemäßen Verbindungen gegebenenfalls mit den entsprechenden (i) Lösungsmitteln und/oder (ii) Säuren oder Basen in ihre Solvate, Salze und/oder Solvate der Salze überführt.

Inerte Lösungsmittel für den Verfahrensschritt (II) + (III) → (I) sind beispielsweise Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Dichlormethan, Trichlormethan, Tetrachlormethan, 1,2-Dichlorethan, Trichlorethylen oder Chlorbenzol, oder andere Lösungsmittel wie Aceton, Ethylacetat, Acetonitril, Pyridin, Dimethylsulfoxid, *N,N*-Dimethylformamid (DMF), *N,N'-*Dimethylpropylenharnstoff (DMPU) oder *N*-Methylpyrrolidon (NMP). Ebenso ist es möglich, Gemische der genannten Lösungsmittel zu verwenden. Bevorzugt wird Dimethylformamid verwendet.

Als Basen für den Verfahrensschritt (II) + (III) → (I) eignen sich die üblichen anorganischen oder organischen Basen. Hierzu gehören bevorzugt Alkalihydroxide wie beispielsweise Lithium-, Natrium- oder Kaliumhydroxid, Alkali- oder Erdalkalicarbonate wie Lithium-, Natrium-, Kalium-oder Cäsiumcarbonat, Alkali-Alkoholate wie Natrium- oder Kaliummethanolat, Natrium- oder Kaliumethanolat oder Lithium-, Natrium- oder Kalium-tert.-butylat, Alkalihydride wie Natrium-oder Kaliumhydrid, Amide wie Natriumamid, Lithium- oder Kalium-bis(trimethylsilyl)amid oder Lithiumdiisopropylamid, oder organische Amine wie *N,N*-Diisopropylethylamin, 1,5-Diazabicyclo[4.3.0]non-5-en (DBN) und 1,8-Diazabicyclo[5.4.0]undec-7-en (DBU). Bevorzugt werden Cäsiumcarbonat und Natriumhydrid verwendet.

Die Base wird hierbei in einer Menge von 1 bis 5 Mol, bevorzugt in einer Menge von 1 bis 2.5 Mol, bezogen auf 1 Mol der Verbindung der Formel (II), eingesetzt. Die Reaktion erfolgt im Allgemeinen in einem Temperaturbereich von -10°C bis +100°C, bevorzugt bei +0°C bis +30°C. Die Umsetzung kann bei normalem, erhöhtem oder bei erniedrigtem Druck durchgeführt werden (z.B. von 0.5 bis 5 bar), gegebenenfalls in einer Mikrowelle. Im Allgemeinen arbeitet man bei Normaldruck.

Die Verbindungen der Formel (II) sind literaturbekannt oder können in Analogie zu literaturbekannten Verfahren ausgehend von kommerziell erhältlichen oder in der Literatur beschriebenen Verbindungen hergestellt werden [vgl. z. B. WO 03/095451 und WO 03/097063]. Die Verbindungen der Formel (III) sind kommerziell erhältlich, literaturbekannt oder in Analogie zu literaturbekannten Verfahren herstellbar.

Die Herstellung der erfindungsgemäßen Verbindungen kann durch die folgenden Syntheseschemata beispielhaft veranschaulicht werden:

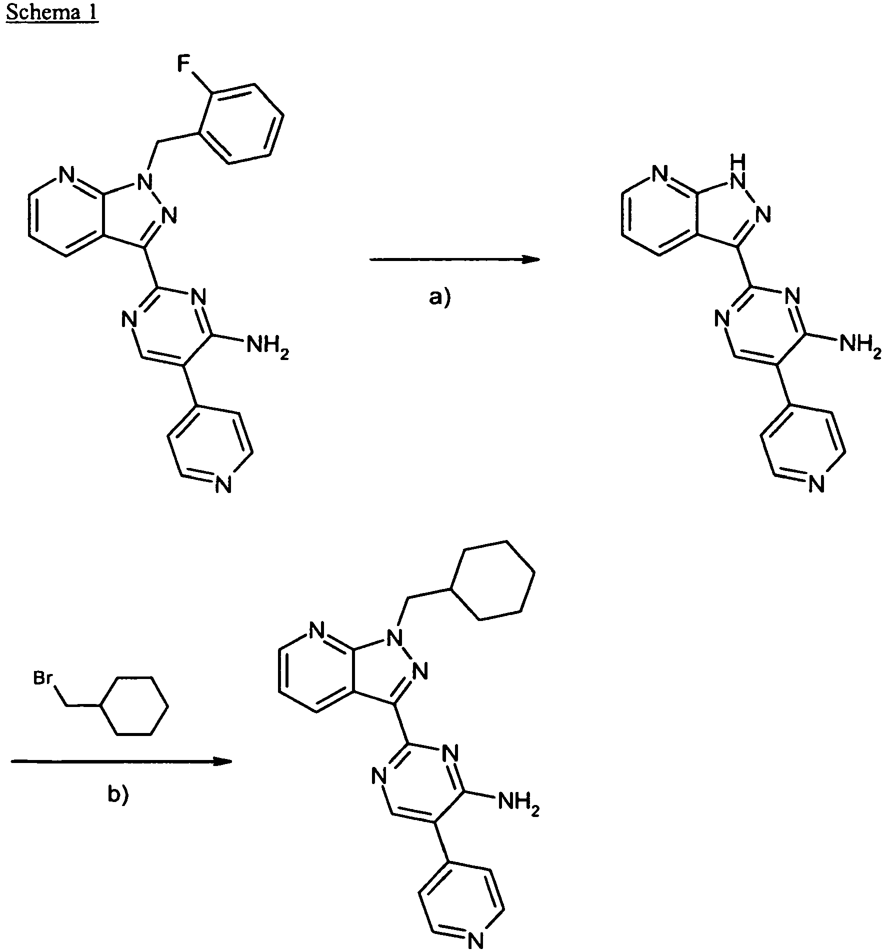

### [a) Natrium, NH₃, NH₄Cl; b) CsCO₃, DMF]

Die erfindungsgemäßen Verbindungen besitzen wertvolle pharmakologische Eigenschaften und können zur Vorbeugung und Behandlung von Erkrankungen bei Menschen und Tieren verwendet werden. Die erfindungsgemäßen Verbindungen eröffnen eine weitere Behandlungsalternative und stellen somit eine Bereicherung der Pharmazie dar.

Die erfindungsgemäßen Verbindungen bewirken eine Gefäßrelaxation und eine Hemmung der Thrombozytenaggregation und führen zu einer Blutdrucksenkung sowie zu einer Steigerung des koronaren Blutflusses. Diese Wirkungen sind über eine direkte Stimulation der löslichen Guanylatcyclase und einen intrazellulären cGMP-Anstieg vermittelt. Außerdem verstärken die erfindungsgemäßen Verbindungen die Wirkung von Substanzen, die den cGMP-Spiegel steigern, wie beispielsweise EDRF (endothelium-derived relaxing factor), NO-Donatoren, Protoporphyrin IX, Arachidonsäure oder Phenylhydrazin-Derivate.

Die erfindungsgemäßen Verbindungen können daher in Arzneimitteln zur Behandlung von kardiovaskulären Erkrankungen wie beispielsweise zur Behandlung des Bluthochdrucks und der Herzinsuffizienz, stabiler und instabiler Angina pectoris, pulmonaler Hypertonie, peripheren und kardialen Gefäßerkrankungen, Arrhythmien, zur Behandlung von thromboembolischen Erkrankungen und Ischämien wie Myokardinfarkt, Hirnschlag, transistorischen und ischämischen Attacken, peripheren Durchblutungsstörungen, Reperfusionsschäden, zur Verhinderung von Restenosen wie nach Thrombolysetherapien, percutan-transluminalen Angioplastien (PTA), percutan-transluminalen Koronarangioplastien (PTCA) und Bypass, sowie zur Behandlung von Arteriosklerose, asthmatischen Erkrankungen, Krankheiten des Urogenitalsystems wie beispielsweise Prostatahypertrophie, erektile Dysfunktion, weibliche sexuelle Dysfunktion und Inkontinenz, von Osteoporose, Glaukom und Gastroparese eingesetzt werden.

Außerdem können die erfindungsgemäßen Verbindungen zur Behandlung von primärem und sekundärem Raynaud-Phänomen, von Mikrozirkulationsstörungen, Claudicatio, peripheren und autonomen Neuropathien, diabetischen Mikroangiopathien, diabetischer Retinopathie, diabetischen Geschwüren an den Extremitäten, Gangren, CREST-Syndrom, Erythematose, Onychomykose, rheumatischen Erkrankungen sowie zur Förderung der Wundheilung verwendet werden.

Weiterhin eignen sich die erfindungsgemäßen Verbindungen zur Behandlung urologischer Erkrankungen wie beispielsweise benignes Prostata-Syndrom (BPS), benigne Prostata-Hyperplasie (BPH), benigne Prostata Vergrösserung (BPE), Blasenentleerungsstörung (BOO), untere Harnwegssyndrome (LUTS), Erkrankungen des Urogenital-Systems einschliesslich neurogene überaktive Blase (OAB) und (IC), Inkontinenz (UI) wie beispielsweise Misch-, Drang-, Stress-, oder Überlauf-Inkontinenz (MUI, UUI, SUI, OUI), Beckenschmerzen, benigne und maligne Erkrankungen der Organe des männlichen und weiblichen Urogenital-Systems, Nierenerkrankungen wie beispielsweise akutes oder chronisches Nierenversagen, immunologische Nierenerkrankungen wie Nierentransplantatabstoßung, Glumerulonephritis, Immunkomplexinduzierte Nierenerkrankungen, Glomerulopathien, Nephritis, toxische Nephropathie und obstruktive Uropathien.

Ferner eignen sich die erfindungsgemäßen Verbindungen zur Behandlung von akuten und chronischen Lungenkrankheiten, wie den Respiratory Distress-Syndromen (ALI, ARDS) und chronisch-obstruktiven Atemwegserkrankungen (COPD), sowie zur Behandlung von akuter und chronischer Niereninsuffizienz.

Die in der vorliegenden Erfindung beschriebenen Verbindungen stellen auch Wirkstoffe zur Bekämpfung von Krankheiten im Zentralnervensystem dar, die durch Störungen des NO/cGMP-Systems gekennzeichnet sind. Insbesondere sind sie geeignet zur Verbesserung der Wahrnehmung, Konzentrationsleistung, Lernleistung oder Gedächtnisleistung nach kognitiven Störungen, wie sie insbesondere bei Situationen/Krankheiten/Syndromen auftreten wie "Mild cognitive impairment", altersassoziierten Lern- und Gedächtnisstörungen, altersassoziierten Gedächtnisverlusten, vaskulärer Demenz, Schädel-Hirn-Trauma, Schlaganfall, Demenz, die nach Schlaganfällen auftritt ("post stroke dementia"), post-traumatischem Schädel-Hirn-Trauma, allgemeinen Konzentrationsstörungen, Konzentrationsstörungen bei Kindern mit Lern- und Gedächtnisproblemen, Alzheimer'scher Krankheit, Demenz mit Lewy-Körperchen, Demenz mit Degeneration der Frontallappen einschliesslich des Pick's-Syndroms, Parkinson'scher Krankheit, progressiver nuclear palsy, Demenz mit corticobasaler Degeneration, Amyolateralsklerose (ALS), Huntington'scher Krankheit, Multipler Sklerose, Thalamischer Degeneration, Creutzfeld-Jacob-Demenz, HIV-Demenz, Schizophrenie mit Demenz oder Korsakoff-Psychose. Sie eignen sich auch zur Behandlung von Erkrankungen des Zentralnervensystems wie Angst-, Spannungs- und Depressionszuständen, zentral-nervös bedingten Sexualdysfunktionen und Schlafstörungen sowie zur Regulierung krankhafter Störungen der Nahrungs-, Genuss- und Suchtmittelaufnahme.

Weiterhin eignen sich die erfindungsgemäßen Verbindungen auch zur Regulation der cerebralen Durchblutung und stellen wirkungsvolle Mittel zur Bekämpfung von Migräne dar. Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen zur Bekämpfung von Schmerzzuständen eingesetzt werden.

Zudem besitzen die erfindungsgemäßen Verbindungen antünflammatorische Wirkung und können daher als entzündungshemmende Mittel eingesetzt werden.

Weiterer Gegenstand der vorliegenden Erfindung sind die erfindungsgemäßen Verbindungen zur Verwendung in einem Verfahren zur Bahndlung und/ oder Prävention von Herzinsuffizienz, Angina pectoris, Hypertonie, pulmonaler Hypertonie, Ischämien, Gefäßerkrankungen, thromboembolischen Erkrankungen und Arteriosklerose.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen. Als geeignete Kombinationswirkstoffe seien beispielhaft und vorzugsweise genannt:
● organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
● Verbindungen, die den Abbau von cyclischem Guanosinmonophosphat (cGMP) inhibieren, wie beispielsweise Inhibitoren der Phosphodiesterasen (PDE) 1, 2 und/oder 5, insbesondere PDE 5-Inhibitoren wie Sildenafil, Vardenafil und Tadalafil;
● antithrombotisch wirkende Mittel, beispielhaft und vorzugsweise aus der Gruppe der Thrombozytenaggregationshemmer, der Antikoagulantien oder der profibrinolytischen Substanzen;
● den Blutdruck senkende Wirkstoffe, beispielhaft und vorzugsweise aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika; und/oder
● den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban (BAY 59-7939), DU-176b, Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embursatan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Diuretikum, wie beispielhaft und vorzugsweise Furosemid, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Torcetrapib (CP-529 414), JJT-705 oder CETP-vaccine (Avant), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin, Cerivastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen:

| | |
|---|---|
| aq. | wässrige Lösung |
| br.s | breites Singulett (bei NMR) |
| DCC | *N,N'-*Dicyclohexylcarbodiimid |
| DMF | Dimethylformamid |
| DMSO | Dimethylsulfoxid |
| d. Th. | der Theorie (bei Ausbeute) |
| eq. | Äquivalent(e) |
| ESI | Elektrospray-Ionisation (bei MS) |
| Et | Ethyl |
| h | Stunde(n) |
| HOAc | Essigsäure |
| HOBt | 1-Hydroxy-1*H-*benzotriazol x H₂O |
| HPLC | Hochdruck-, Hochleistungsflüssigchromatographie |
| iPr | Isopropyl |
| LC/MS | Flüssigchromatographie-gekoppelte Massenspektrometrie |
| LDA | Lithiumdiisopropylamid |
| min | Minute(n) |
| MS | Massenspektrometrie |
| NMR | Kernresonanzspektrometrie |
| RT | Raumtemperatur |
| Rₜ | Retentionszeit (bei HPLC) |
| THF | Tetrahydrofuran |
| UV | Ultraviolett-Spektrometrie |
| v/v | Volumen zu Volumen-Verhältnis (einer Lösung) |

### LC/MS- und HPLC-Methoden:

### Methode 1 (LC/MS):

Instrument: Micromass Quattro LCZ mit HPLC Agilent Serie 1100; Säule: Phenomenex Synergi 2µ Hydro-RP Mercury 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 208-400 nm.

### Methode 2 (LC/MS):

Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 3 (LC/MS):

Instrument: Micromass Platform LCZ mit HPLC Agilent Serie 1100; Säule: Thermo Hypersil GOLD 3p 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 100% A → 0.2 min 100% A → 2.9 min 30% A → 3.1 min 10% A → 5.5 min 10% A; Ofen: 50°C; Fluss: 0.8 ml/min; UV-Detektion: 210 nm.

### Methode 4 (präparative HPLC):

Säule: Kromasil 100 C 18, 5 µm, 250 mm x 20 mm; Eluent A: 0.2% Trifluoressigsäure in Wasser, Eluent B: Acetonitril; A : B 60 : 40 isokratisch; Fluss: 25 ml/min; Injektionsvolumen 500 µl, Temperatur 30 °C.

### Methode 5 (präparative HPLC):

Säule: Grom-Sil 120 ODS-4HE, 10 µm, 250 mm x 30 mm; Eluent A: 0.1% Ameisensäure in Wasser, Eluent B: Acetonitril; Fluss: 50 ml/min; Gradient: 0-3 min 10% B, 3-27 min 10% → 95% B, 27-34 min 95% B, 34-38 min 10% B.

### Ausgangsverbindung

### Beispiel 1A

Methyl-[4,6-diamino-2-(1H-pyrazolo[3,4-b]pyridin-3-yl)pyrimidin-5-yl]carbamat

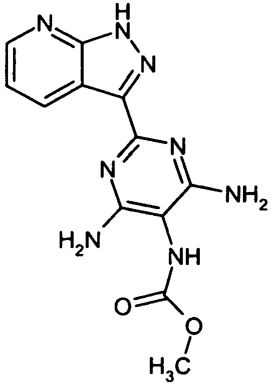

In einem 1L-Kolben wurden unter Trockeneiskühlung 540 ml Ammoniak kondensiert. Man gab portionsweise bei ca. -50°C 8.1 g (352.6 mmol) Natrium zu und ließ 0.5 h bei -40°C rühren. Dann wurden 36.0 g (88.1 mmol) Methyl-{4,6-diamino-2-[1-(2-fluorobenzyl)-1H-pyrazolo[3,4-b]pyridin-3-yl]pyrimidin-5-yl}carbamat (Herstellung: siehe WO 03/095451, Beispiel 5) zugegeben. Die Mischung wurde 5 h bei der gleichen Temperatur gerührt, dann mit 28.3 g (528.9 mmol) Ammoniumchlorid versetzt und 0.5 h weiter bei -40°C gerührt. Danach wurde die Kühlung entfernt und die Mischung ohne Rühren über Nacht stehen gelassen, damit Ammoniak verdampft. Zum Rückstand wurden 750 ml Wasser zugegeben. Es wurde 0.5 h gerührt, dann wurde der Feststoff abgesaugt und in Vakuum getrocknet.

Dieses Rohprodukt (34 g) wurde in einer Mischung aus 200 ml Methanol, 50 ml THF und 50 ml Wasser gelöst. Die Lösung wurde mit Trifluoressigsäure auf pH 2 gestellt und portionsweise mittels HPLC (Methode 4) getrennt. Man erhielt insgesamt 11.3 g zurückgewonnenes Edukt sowie 17.8 g (51 % d. Th.) einer produkthaltigen Fraktion (76%ige Reinheit (LC-MS)).
1 g dieser Fraktion wurde durch präparative HPLC (Methode 5) erneut aufgereinigt. Man erhielt 116 mg Produkt in guter Reinheit (ca. 8 % d. Th.).
¹H-NMR (400 MHz, DMSO-*d₆*): δ = 14.8 (br.s, 1H), 13.3-12.5 (br.s, 2 H), 8.91 (d, *J* = 8.1 Hz, 1H), 8.71 (d, *J* = 4.7 Hz, 1H), 8.34 (s, 1H), 7.46 *(dd, J=* 8.1, 4.7 Hz, 1H), 3.66 (s, 3H).
LC-MS (Methode 3): Rₜ = 2.11 Min.; MS (ESIpos): m/z = 301 [M+H]⁺.

### Ausführungsbeispiele

### Beispiel 1

Methyl-[4,6-diamino-2-(1-cyclohexylrnethyl-1H-pyrazolo[3,4-b]pyridin-3-yl)pyrimidin-5-yl]carbamat

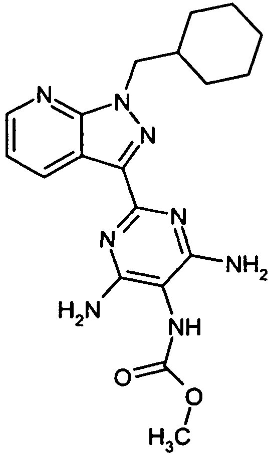

330 mg (1.1 mmol) der Verbindung aus Beispiel 1A und 212 mg (1.2 mmol) Brommethylcyclohexan wurden in 5 ml Dimethylformamid vorgelegt, mit 389 mg (1.2 mmol) Cäsiumcarbonat versetzt und die resultierende Mischung über Nacht bei RT verrührt. Es wurden 0.5 ml 1N Salzsäure zugegeben und die gesamte Lösung über HPLC (Methode 5) getrennt. Die entsprechenden Fraktionen wurden am Rotationsverdampfer von den flüchtigen Komponenten befreit und der Rückstand am Hochvakuum getrocknet. Man erhielt 60 mg (19 % d. Th.) der Zielverbindung.

¹H-NMR (500 MHz, DMSO-*d₆*): δ = 9.03 (d, 1H), 8.55 (d, 1H), 8.04 (s, 1H), 7.28 (dd, 1H), 6.16 (br. s, 4H), 4.35 (d, 2H), 3.63 (s, 3H), 2.00 (m, 1H), 1.69-1.46 (m, 5H), 1.19-1.10 (m, 3H), 1.08-0.98 (m, 2H).

LC-MS (Methode 1): Rₜ = 1.61 Min.; MS (ESIpos): m/z = 397 [M+H]⁺.

### Beispiel 2

Methyl-[4,6-diamino-2-(1-cycloheptylmethyl-1H-pyrazolo[3,4-b]pyridin-3-yl)pyrimidin-5-yl]-carbamat

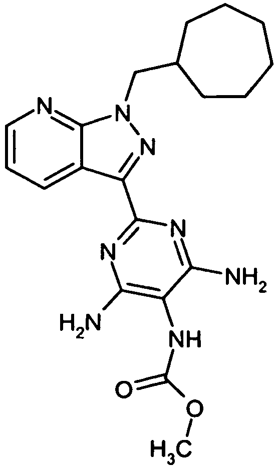

300 mg (1.0 mmol) der Verbindung aus Beispiel 1A und 224 mg (1.1 mmol) CycloheptylmethylMethansulfonat (Herstellung: siehe J. Med. Chem. 2000, 43(26), 5017-5029) wurden in 5 ml Dimethylformamid vorgelegt, mit 354 mg (1.1 mmol) Cäsiumcarbonat versetzt und die resultierende Mischung über Nacht bei RT verrührt. Es wurden 0.5 ml 1N Salzsäure zugegeben und die gesamte Lösung über HPLC (Methode 5) getrennt. Die entsprechenden Fraktionen wurden am Rotationsverdampfer eingeengt und der Rückstand am Hochvakuum getrocknet. Man erhielt 2.8 mg (0.7 % d. Th.) der Zielverbindung.

¹H-NMR (400 MHz, CDCl₃): δ = 8.82 (d, 1H), 8.55 (d, 1H), 8.14 (br. s, 1H), 7.19 (dd, 1H), 6.0 (br. S, 1H), 5.35-5.05 (br. s, 3H), 4.44 (d, 2H), 3.79 (s, 3H), 2.42 (m, 1H), 1.69-1.21 (m, 12H).

LC-MS (Methode 1): Rₜ = 1.80 Min.; MS (ESIpos): m/z = 411 [M+H]⁺.

### Beispiel 3

Methyl-N-[4,6-diamino-2-(1-cyclohexylmethyl-1H-pyrazolo[3,4-b]pyridin-3-yl)pyrimidin-5-yl]-N-methyl-carbamat

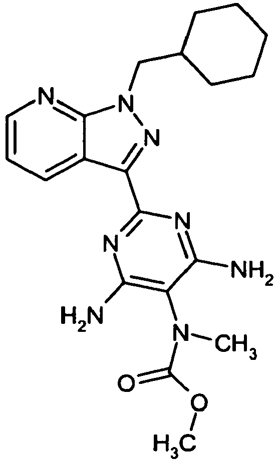

24 mg (61 µmol) der Verbindung aus Beispiel 1 wurden in 1 ml Dimethylformamid vorgelegt und mit ca. 2.7 mg (70 µmol) Natriumhydrid (60 % in Mineralöl) versetzt. Die resultierende Mischung wurde 10 min bei RT verrührt, dann wurden 6 µl (9 µmol) Iodmethan zugegeben und die Reaktion über Nacht gerührt. Es wurden 0.5 ml 1N Salzsäure zugegeben und die gesamte Mischung über HPLC (Methode 5) getrennt. Die entsprechenden Fraktionen wurden am Rotationsverdampfer eingeengt und der Rückstand im Hochvakuum getrocknet. Man erhielt 8 mg (32 % d. Th.) der Zielverbindung.

¹H-NMR (500 MHz, CDCl₃): δ = 8.88 (dd, 1H), 8.55 (dd, 1H), 7.20 (dd, 1H), 4.84 (br s, 4H), 4.49 (d, 2H), 3.70 (br s, 3H), 3.20 (s, 3H), 2.20 (m, 1H), 1.69-1.60 (m, 3H), 1.56-1.51 (m, 2H), 1.22-1.03 (m, 5H).

LC-MS (Methode 2): Rₜ = 1.62 Min.; MS (ESIpos): m/z = 411 [M+H]⁺.

### Beispiel 4

2-[1-(Cyclohexylmethyl)-1H-pyrazolo[3,4-b]pyridin-3-yl]-5-(pyridin-4-yl)pyrimidin-4-amin

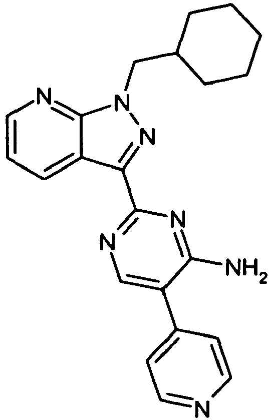

200 mg (0.691 mmol) 2-(1H-Pyrazolo[3,4-b]pyridin-3-yl)-5-(pyridin-4-yl)pyrimidin-4-amin (Herstellung: siehe WO 03/097063, Beispiel 35A) wurden in 5 ml DMF gelöst und mit 248 mg (0.760 mmol) Cäsiumcarbonat sowie 136 mg (0.760 mmol) Cyclohexylmethylbromid versetzt. Das Gemisch wurde über Nacht bei RT gerührt. Es wurde Wasser addiert und dreimal mit Dichlormethan extrahiert. Die vereinigten organischen Phasen wurden mit gesättigter wässriger Natriumchlorid-Lösung gewaschen, über Natriumsulfat getrocknet und am Rotationsverdampfer eingeengt. Der Rückstand wurde mittels präparativer HPLC (Methode 5) fraktioniert. Die produkthaltigen Fraktionen wurden vereinigt, am Rotationsverdampfer eingeengt und der Rückstand im Hochvakuum getrocknet. Es wurden 29 mg (11% d. Th.) der Zielverbindung erhalten.

¹H-NMR (500 MHz, CDCl*₃*): δ = 1.08-1.25 (m, 5H), 1.56-1.71 (m, 5H), 2.20-2.28 (m, 1H), 4.51 (d, 2H), 5.33 (s br, 2H), 7.25 (dd, 1H), 7.46 (d, 2H), 8.40 (s, 1H), 8.59 (d, 1H), 8.77 (s br, 2H), 8.94 (d, 1H).

LC-MS (Methode 1): Rₜ = 1.84 Min.; MS (ESIpos): m/z = 386 [M+H]⁺.

### Beispiel 5

2-[1-(Cycloheptylmethyl)-1H-pyrazolo[3,4-b]pyridin-3-yl]-5-(pyridin-4-yl)pyrimidin-4-amin

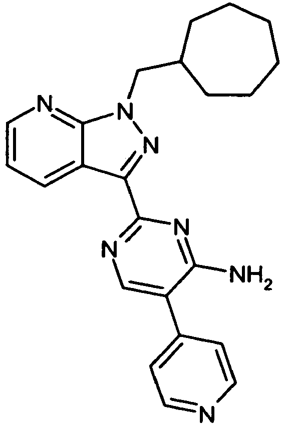

200 mg (0.691 mmol) 2-(1H-Pyrazolo[3,4-b]pyridin-3-yl)-5-(pyridin-4-yl)pyrimidin-4-amin (Herstellung: siehe WO 03/097063, Beispiel 35A) wurden in 4 ml DMF gelöst und mit 451 mg (1.383 mmol) Cäsiumcarbonat sowie 288 mg (1.383 mmol) Cycloheptylmethylmethansulfonat (Herstellung: siehe WO 96/05193, S. 147) versetzt. Das Gemisch wurde über Nacht bei RT gerührt. Es wurde Wasser addiert und dreimal mit Dichlormethan extrahiert. Die vereinigten organischen Phasen wurden mit gesättigter wässriger Natriumchlorid-Lösung gewaschen, über Natriumsulfat getrocknet und am Rotationsverdampfer eingeengt. Der Rückstand wurde mittels präparativer HPLC (Methode 5) fraktioniert. Die produkthaltigen Fraktionen wurden vereinigt und am Rotationsverdampfer eingeengt. Der Rückstand wurde im Hochvakuum getrocknet. Es wurden 45 mg (16% d. Th.) der Zielverbindung erhalten.

¹H-NMR (500 MHz, DMSO-*d₆*): δ = 1..21-1.66 (m, 12H), 2.24-2.34 (m, 1H), 4.40 (d, 2H), 7.17 (s br, 2H), 7.36 (dd, 1H), 7.56 (d, 2H), 8.29 (s, 1H), 8.61 (dd, 1H), 8.68 (d, 2H), 9.01 (dd, 1H).

LC-MS (Methode 1): Rₜ = 1.97 Min.; MS (ESIpos): m/z = 400 [M+H]⁺.

### B. Bewertung der pharmakologischen Wirksamkeit

Die pharmakologische Wirkung der erfindungsgemäßen Verbindungen kann in folgenden Assays gezeigt werden:

### B-1. Gefäßrelaxierende Wirkung in vitro

Kaninchen werden durch Nackenschlag betäubt und entblutet. Die Aorta wird entnommen, von anhaftendem Gewebe befreit, in 1.5 mm breite Ringe geteilt und einzeln unter einer Vorspannung in 5 ml-Organbäder mit 37°C warmer, Carbogen-begaster Krebs-Henseleit-Lösung folgender Zusammensetzung gebracht (jeweils mM): NaCl: 119; KCI: 4.8; CaCl₂ x 2 H₂O: 1; MgSO₄ x 7 H₂O: 1.4; KH₂PO₄: 1.2; NaHCO₃: 25; Glucose: 10. Die Kontraktionskraft wird mit Statham UC2-Zellen erfasst, verstärkt und über A/D-Wandler (DAS-1802 HC, Keithley Instruments München) digitalisiert sowie parallel auf Linienschreiber registriert. Zur Erzeugung einer Kontraktion wird Phenylephrin dem Bad kumulativ in ansteigender Konzentration zugesetzt. Nach mehreren Kontrollzyklen wird die zu untersuchende Substanz in jedem weiteren Durchgang in jeweils steigender Dosierung zugesetzt und die Höhe der Kontraktion mit der Höhe der im letzten Vordurchgang erreichten Kontraktion verglichen. Daraus wird die Konzentration errechnet, die erforderlich ist, um die Höhe des Kontrollwertes um 50% zu reduzieren (IC₅₀-Wert). Das Standardapplikationsvolumen beträgt 5 µl, der DMSO-Anteil in der Badlösung entspricht 0.1%.

Repräsentative IC₅₀-Werte für die erfindungsgemäßen Verbindungen sind in der nachstehenden Tabelle wiedergegeben:

| **Beispiel Nr.** | **IC₅₀ [nM]** |
|---|---|
| 1 | 870 |
| 5 | 1000 |

### B-2. Wirkung an rekombinanter Guanylatcyclase-Reporterzelllinie

Die zelluläre Wirkung der erfindungsgemäßen Verbindungen wird an einer rekombinanten Guanylatcyclase-Reporterzelllinie, wie in F. Wunder et al., Anal. Biochem. 339, 104-112 (2005) beschrieben, bestimmt.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v:-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 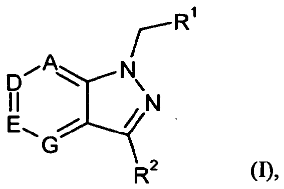 in welcher
A für CR³ oder N steht,
wobei
R³ für Wasserstoff, Halogen, Cyano, (C₁-C₄)-Alkyl, Trifluormethyl, Amino, (C₁-C₄)-Alkoxy und Trifluormethoxy steht,
D für CR⁴ oder N steht,
wobei
R⁴ für Wasserstoff, Halogen, Cyano, (C₁-C₄)-Alkyl, Trifluormethyl, Amino, (C₁-C₄)-Alkoxy und Trifluormethoxy steht,
E für CR⁵ oder N steht,
wobei
R⁵ für Wasserstoff, Halogen, Cyano, (C₁-C₄)-Alkyl, Trifluormethyl, Amino, (C₁-C₄)-Alkoxy und Trifluormethoxy steht,
G für CR⁶ oder N steht,
wobei
R⁶ für Wasserstoff, Halogen, Cyano, (C₁-C₄)-Alkyl, Trifluormethyl, Amino, (C₁-C₄)-Alkoxy und Trifluormethoxy steht,
mit der Maßgabe, dass maximal 2 der Gruppen A, D, E und G für N stehen,
R¹ für (C₃-C₈)-Cycloalkyl steht,
wobei (C₃-C₈)-Cycloalkyl mit 1 oder 2 Substituenten unabhängig voneinander ausgewählt aus der Gruppe Fluor und (C₁-C₄)-Alkyl substituiert sein kann,
R² für Pyrrolinyl, Dihydropyrazolyl, Imidazolinyl, Dihydrooxazolyl, Dihydroisoxazolyl, Dihydro-1,2,4-triazolyl, Dihydro-1,2,4-oxadiazolyl, Dihydro-1,3,4-oxadiazolyl, Dihydro-1,2,4-thiadiazolyl, Dihydropyranyl, 1,4-Dihydropyridyl, Tetrahydropyrimidinyl, 1,3-Oxazinyl, Furyl, Thienyl, Thiazolyl, Oxazolyl, Isothiazolyl, Isoxazolyl, Pyrazolyl, Imidazolyl, Triazolyl, Oxadiazolyl, Thiadiazolyl, Pyridyl, Pyrimidinyl, Pyridazinyl, Pyrazinyl oder Triazinyl steht,
wobei Pyrrolinyl, Dihydropyrazolyl, Imidazolinyl, Dihydrooxazolyl, Dihydroisoxazolyl, Dihydro-1,2,4-triazolyl, Dihydro-1,2,4-oxadiazolyl, Dihydro-1,3,4-oxadiazolyl, Dihydro-1,2,4-thiadiazolyl, Dihydropyranyl, 1,4-Dihydropyridyl, Tetrahydropyrimidinyl, 1,3-Oxazinyl, Furyl, Thienyl, Thiazolyl, Oxazolyl, Isothiazolyl, Isoxazolyl, Pyrazolyl, Imidazolyl, Triazolyl, Oxadiazolyl, Thiadiazolyl, Pyridyl, Pyrimidinyl, Pyridazinyl, Pyrazinyl und Triazinyl mit 1 bis 4 Substituenten unabhängig voneinander ausgewählt aus der Gruppe Halogen, Azido, Nitro, Cyano, -R⁷, -C(=O)-R⁷, -C(=O)-OR⁷, -C(=O)-NR⁷R⁸, -O-(C=O)ₙ-R⁷, -O-C(=O)-OR⁷, -O-C(=O)-NR⁷R⁸, -S(O)ₚ-R⁷, -SO₂-OR⁷, -SO₂-NR⁷R⁸, -NR⁷-(C=O)ₙ-R⁸, -NR⁷-SO₂-R⁸, -NR⁷-C(=O)-OR⁸, -NR⁹-C(=O)-NR⁷R⁸ und -NR⁹-SO₂-NR⁷R⁸ substituiert sein kann,
worin
n für eine Zahl 0 oder 1 steht,
p für eine Zahl 0, 1 oder 2 steht,
R⁷, R⁸ und R⁹ jeweils unabhängig voneinander für Wasserstoff, (C₁-C₆)-Alkyl, (C₂-C₆)-Alkenyl, (C₃-C₈)-Cycloalkyl, (C₃-C₈)-Cycloalkenyl, (C₆-C₁₀)-Aryl, 4- bis 8-gliedriges Heterocyclyl oder 5- bis 10-gliedriges Heteroaryl stehen,
worin R⁷, R⁸ und R⁹ ihrerseits mit 1 bis 5 Substituenten unabhängig voneinander ausgewählt aus der Gruppe Halogen, Azido, Nitro, Cyano, Trifluormethyl, (C₁-C₆)-Alkyl, (C₁-C₆)-Alkylcarbonyl, (C₁-C₆)-Alkylcarbonyloxy, Hydroxycarbonyl, (C₁-C₆)-Alkoxycarbonyl, Aminocarbonyl, Mono-(C₁-C₆)-alkylaminocarbonyl, Di-(C₁-C₆)-alkylaminocarbonyl, Hydroxy, Trifluormethoxy, (C₁-C₆)-Alkoxy, Oxo, Mercapto, (C₁-C₆)-Alkylthio, Amino, Mono-(C₁-C₆)-alkylamino, Di-(C₁-C₆)-alkylamino, Formylamino, (C₁-C₆)-Alkylcarbonylamino, (C₁-C₆)-Alkoxycarbonylamino, (C₃-C₈)-Cycloalkyl, (C₃-C₈)-Cycloalkenyl sowie 4- bis 8-gliedriges Heterocyclyl substituiert sein können,
oder
R⁷ und R⁸ zusammen mit dem Rest, an den sie jeweils beide gebunden sind, einen 4- bis 8-gliedrigen Heterocyclus bilden,
oder
R⁷ und R⁹ zusammen mit dem Rest, an den sie jeweils beide gebunden sind, einen 4- bis 8-gliedrigen Heterocyclus bilden,
sowie ihre *N*-Oxide, Salze, Solvate, Salze der *N*-Oxide und Solvate der *N*-Oxide und Salze.

2. Verbindung der Formel (I) nach Anspruch 1, in welcher
A für CR³ oder N steht,
wobei
R³ für Wasserstoff steht,
D für CR⁴ oder N steht,
wobei
R⁴ für Wasserstoff steht,
E für CR⁵ oder N steht,
wobei
R⁵ für Wasserstoff steht,
G für CR⁶ oder N steht,
wobei
R⁶ für Wasserstoff steht,
mit der Maßgabe, dass maximal 2 der Gruppen A, D, E und G für N stehen,
R¹ für Cyclopentyl, Cyclohexyl oder Cycloheptyl steht,
wobei Cyclopentyl, Cyclohexyl oder Cycloheptyl mit 1 oder 2 Substituenten unabhängig voneinander ausgewählt aus der Gruppe Fluor und Methyl substituiert sein können,
R² für eine Gruppe der Formel 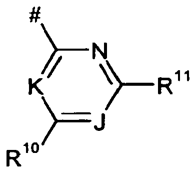 steht,
wobei
# für die Anknüpfstelle an den Heterobicyclus steht,
K für CH oder N steht,
J für CR¹², N oder N⁺-O⁻ steht,
worin
R¹² für Halogen, Nitro, Cyano, -R⁷, -C(=O)-R⁷, -C(=O)-OR⁷, -C(=O)-NR⁷R⁸, -O-(C=O)ₙ-R⁷, -O-C(=O)-OR⁷, -O-C(=O)-NR⁷R⁸, -S(O)ₚ-R⁷, -SO₂-OR⁷, -SO₂-NR⁷R⁸, -NR⁷-(C=O)ₙ-R⁸, -NR⁷-SO₂-R⁸, -NR⁷-C(=O)-OR⁸, -NR⁹-C(=O)-NR⁷R⁸ und -NR⁹-SO₂-NR⁷R⁸ steht,
worin
n die Zahl 0 oder 1 darstellt,
p die Zahl 0 oder 2 darstellt,
R⁷, R⁸ und R⁹ jeweils unabhängig voneinander für Wasserstoff, (C₁-C₆)-Alkyl, (C₂-C₆)-Alkenyl, (C₃-C₇)-Cycloalkyl, (C₃-C₇)-Cycloalkenyl, Phenyl, 5- bis 7-gliedriges Heterocyclyl oder 5- oder 6-gliedriges Heteroaryl stehen,
worin R⁷, R⁸ und R⁹ ihrerseits mit 1 bis 3 Substituenten unabhängig voneinander ausgewählt aus der Gruppe Fluor, Chlor, Cyano, Trifluormethyl, (C₁-C₄)-Alkyl, Hydroxy, Trifluormethoxy, (C₁-C₄)-Alkoxy, Oxo, Amino, Mono-(C₁-C₄)-alkylamino und Di-(C₁-C₄)-alkylamino substituiert sein können,
oder
R⁷ und R⁸ zusammen mit dem Rest, an den sie jeweils beide gebunden sind, einen 5- bis 7-gliedrigen Heterocyclus bilden,
oder
R⁷ und R⁹ zusammen mit dem Rest, an den sie jeweils beide gebunden sind, einen 5- bis 7-gliedrigen Heterocyclus bilden,
sowie ihre *N*-Oxide, Salze, Solvate, Salze der *N*-Oxide und Solvate der *N*-Oxide und Salze.

3. Verbindung der Formel (I) nach Anspruch 1 oder 2, in welcher
A für N steht,
D für CR⁴ steht,
wobei
R⁴ für Wasserstoff steht,
E für CR⁵ steht,
wobei
R⁵ für Wasserstoff steht,
G für CR⁶ steht,
wobei
R⁶ für Wasserstoff steht,
R¹ für Cyclohexyl oder Cycloheptyl steht,
R² für eine Gruppe der Formel 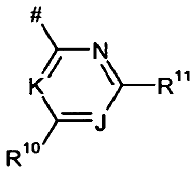
steht,
wobei
# für die Anknüpfstelle an den Heterobicyclus steht,
K für N steht,
J für CR¹² oder N steht,
worin
R¹² für Wasserstoff, -R⁷, -NR⁷-(C=O)ₙ-R⁸, -NR⁷-C(=O)-OR⁸ oder Pyridyl steht,
worin
n für die Zahl 1 steht,
R⁷ für Wasserstoff, Trifluormethyl oder (C₁-C₄)-Alkyl steht, worin (C₁-C₄)-Alkyl seinerseits mit einem Substituenten ausgewählt aus der Gruppe Fluor, Trifluormethyl, Hydroxy und Methoxy substituiert sein kann,
R⁸ für (C₁-C₄)-Alkyl oder (C₃-C₇)-Cycloalkyl steht,
worin (C₁-C₄)-Alkyl und (C₃-C₇)-Cycloalkyl ihrerseits mit einem Substituenten ausgewählt aus der Gruppe Fluor, Trifluormethyl, (C₁-C₄)-Alkyl, Hydroxy, Methoxy und Oxo substituiert sein können,
oder
R⁷ und R⁸ zusammen mit dem Rest, an den sie jeweils beide gebunden sind, einen 5- bis 7-gliedrigen Heterocyclus bilden,
sowie ihre *N*-Oxide, Salze, Solvate, Salze der *N*-Oxide und Solvate der *N*-Oxide und Salze.

4. Verfahren zur Herstellung von Verbindungen der Formel (I), wie in den Ansprüchen 1 bis 3 definiert, **dadurch gekennzeichnet, dass** man eine Verbindung der Formel (II) 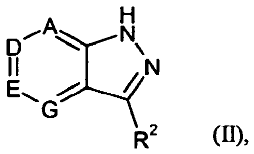 in welcher A, D, E, G und R² jeweils die in den Ansprüchen 1 bis 3 angegebenen Bedeutungen haben,
in einem inerten Lösungsmittel in Gegenwart einer geeigneten Base mit einer Verbindung der Formel (III) 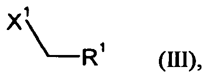 in welcher R¹ die in den Ansprüchen 1 bis 3 angegebene Bedeutung hat
und
X¹ für eine geeignete Abgangsgruppe, wie Tosylat, Mesylat oder Halogen, insbesondere Brom, steht,
umsetzt,
gegebenenfalls die resultierende Verbindung der Formel (I) nach literaturüblichen Verfahren weiter im oben angegebenen Bedeutungsumfang der einzelnen Substituenten und Reste modifiziert
und die resultierenden Verbindungen der Formel (I) gegebenenfalls mit den entsprechenden (i) Lösungsmitteln und/oder (ii) Basen oder Säuren in ihre Solvate, Salze und/oder Solvate der Salze überführt.

5. Verbindung der Formel (I), wie in einem der Ansprüche 1 bis 3 definiert, zur Behandlung und/oder Prophylaxe von Krankheiten.

6. Verbindung der Formel (I), wie in einem der Ansprüche 1 bis 3 definiert, zur Verwendung in einem Verfahren zur Bahndlung und/ oder Prävention von Herzinsuffizienz, Angina pectoris, Hypertonie, pulmonaler Hypertonie, Ischämien, Gefäßerkrankungen, thromboembolischen Erkrankungen und Arteriosklerose.

7. Verwendung einer Verbindung der Formel (I), wie in einem der Ansprüche 1 bis 3 definiert, zur Herstellung eines Arzneimittels zur Behandlung und/oder Prävention von Herzinsuffizienz, Angina pectoris, Hypertonie, pulmonaler Hypertonie, Ischämien, Gefäßerkrankungen, thromboembolischen Erkrankungen und Arteriosklerose.

8. Arzneimittel enthaltend eine Verbindung der Formel (I), wie in einem der Ansprüche 1 bis 3 definiert, in Kombination mit einem inerten, nicht-toxischen, pharmazeutisch geeigneten Hilfsstoff.

9. Arzneimittel enthaltend eine Verbindung der Formel (I), wie in einem der Ansprüche 1 bis 3 definiert, in Kombination mit einem weiteren Wirkstoff ausgewählt aus der Gruppe bestehend aus organischen Nitraten, NO-Donatoren, cGMP-PDE-Inhibitoren, antithrombotisch wirkenden Mitteln, den Blutdruck senkenden Mitteln sowie den Fettstoffwechsel verändernden Mitteln.

10. Arzneimittel nach Anspruch 8 oder 9 zur Behandlung und/oder Prävention von Herzinsuffizienz, Angina pectoris, Hypertonie, pulmonaler Hypertonie, Ischämien, Gefäßerkrankungen, thromboembolischen Erkrankungen und Arteriosklerose.

## Claims

1. Compound of the formula (I) 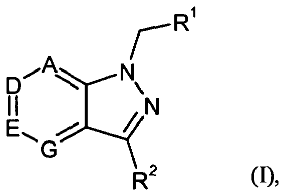 in which
A represents CR³ or N,
where
R³ represents hydrogen, halogen, cyano, (C₁-C₄)-alkyl, trifluoromethyl, amino, (C₁-C₄)-alkoxy or trifluoromethoxy,
D represents CR⁴ or N,
where
R⁴ represents hydrogen, halogen, cyano, (C₁-C₄)-alkyl, trifluoromethyl, amino, (C₁-C₄)-alkoxy or trifluoromethoxy,
E represents CR⁵ or N,
where
R⁵ represents hydrogen, halogen, cyano, (C₁-C₄)-alkyl, trifluoromethyl, amino, (C₁-C₄)-alkoxy or trifluoromethoxy,
G represents CR⁶ or N,
where
R⁶ represents hydrogen, halogen, cyano, (C₁-C₄)-alkyl, trifluoromethyl, amino, (C₁-C₄)-alkoxy or trifluoromethoxy,
with the proviso that at most 2 of the groups A, D, E and G represent N,
R¹ represents (C₃-C₈)-cycloalkyl,
where (C₃-C₈)-cycloalkyl may be substituted by 1 or 2 substituents independently of one another selected from the group consisting of fluorine and (C₁-C₄)-alkyl,
R² represents pyrrolinyl, dihydropyrazolyl, imidazolinyl, dihydrooxazolyl, dihydroisoxazolyl, dihydro-1,2,4-triazolyl, dihydro-1,2,4-oxadiazolyl, dihydro-1,3,4-oxadiazolyl, dihydro-1,2,4-thiadiazolyl, dihydropyranyl, 1,4-dihydropyridyl, tetrahydropyrimidinyl, 1,3-oxazinyl, furyl, thienyl, thiazolyl, oxazolyl, isothiazolyl, isoxazolyl, pyrazolyl, imidazolyl, triazolyl, oxadiazolyl, thiadiazolyl, pyridyl, pyrimidinyl, pyridazinyl, pyrazinyl or triazinyl,
where pyrrolinyl, dihydropyrazolyl, imidazolinyl, dihydrooxazolyl, dihydroisoxazolyl, dihydro-1,2,4-triazolyl, dihydro-1,2,4-oxadiazolyl, dihydro-1,3,4-oxadiazolyl, dihydro-1,2,4-thiadiazolyl, dihydropyranyl, 1,4-dihydropyridyl, tetrahydropyrimidinyl, 1,3-oxazinyl, furyl, thienyl, thiazolyl, oxazolyl, isothiazolyl, isoxazolyl, pyrazolyl, imidazolyl, triazolyl, oxadiazolyl, thiadiazolyl, pyridyl, pyrimidinyl, pyridazinyl, pyrazinyl and triazinyl may be substituted by 1 to 4 substituents independently of one another selected from the group consisting of halogen, azido, nitro, cyano, -R⁷, -C(=O)-R⁷, -C(=O)-OR⁷, -C(=O)-NR⁷R⁸, -O-(C=O)ₙ-R⁷, -O-C(=O)-OR⁷, -O-C(=O)-NR⁷R⁸, -S(O)ₚ-R⁷, -SO₂-OR⁷, - SO₂-NR¹R⁸, -NR⁷-(C=O)ₙ-R⁸, -NR⁷-SO₂-R⁸, -NR⁷-C(=O)-OR⁸, -NR⁹-C(=O)-NR⁷R⁸ and -NR⁹-SO₂-NR⁷R⁸,
in which
n represents a number 0 or 1,
p represents a number 0, 1 or 2,
R⁷, R⁸ and R⁹ each independently of one another represent hydrogen, (C₁-C₆)-alkyl, (C₂-C₆)-alkenyl, (C₃-C₈)-cycloalkyl, (C₃-C₈)-cycloalkenyl, (C₆-C₁₀)-aryl, 4-to 8-membered heterocyclyl or 5- to 10-membered heteroaryl,
in which R⁷, R⁸ and R⁹ for their part may be substituted by 1 to 5 substituents independently of one another selected from the group consisting of halogen, azido, nitro, cyano, trifluoromethyl, (C₁-C₆)-alkyl, (C₁-C₆)-alkylcarbonyl, (C₁-C₆)-alkylcarbonyloxy, hydroxycarbonyl, (C₁-C₆)-alkoxycarbonyl, aminocarbonyl, mono-(C₁-C₆)-alkylaminocarbonyl, di-(C₁-C₆)-alkylaminocarbonyl, hydroxyl, trifluoromethoxy, (C₁-C₆)-alkoxy, oxo, mercapto, (C₁-C₆)-alkylthio, amino, mono-(C₁-C₆)-alkylamino, di-(C₁-C₆)-alkylamino, formylamino, (C₁-C₆)-alkylcarbonylamino, (C₁-C₆)-alkoxycarbonylamino, (C₃-C₈)-cycloalkyl, (C₃-C₈)-cycloalkenyl and 4- to 8-membered heterocyclyl,
or
R⁷ and R⁸ together with the radical to which the two are attached form a 4- to 8-membered heterocycle,
or
R⁷ and R⁹ together with the radical to which the two are attached form a 4- to 8-membered heterocycle,
and also *N*-oxides, salts, solvates, salts of the *N*-oxides and solvates of the *N*-oxides and salts thereof.

2. Compound of the formula (I) according to claim 1 in which
A represents CR³ or N,
where
R³ represents hydrogen,
D represents CR⁴ or N,
where
R⁴ represents hydrogen,
E represents CR⁵ or N,
where
R⁵ represents hydrogen,
G represents CR⁶ or N,
where
R⁶ represents hydrogen,
with the proviso that at most 2 of the groups A, D, E and G represent N,
R¹ represents cyclopentyl, cyclohexyl or cycloheptyl,
where cyclopentyl, cyclohexyl or cycloheptyl may be substituted by 1 or 2 substituents independently of one another selected from the group consisting of fluorine and methyl,
R² represents a group of the formula 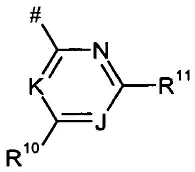 where
# represents the point of attachment to the heterobicycle,
K represents CH or N,
J represents CR¹², N or N⁺-O⁻,
in which
R¹² represents halogen, nitro, cyano, -R⁷, -C(=O)-R⁷, -C(=O)-OR⁷, -C(=O)-NR⁷R⁸, -O-(C=O)ₙ-R⁷, -O-C(=O)-OR⁷, -O-C(=O)-NR⁷R⁸, -S(O)ₚ-R⁷, -SO₂-OR⁷, -SO₂-NR⁷R⁸, -NR⁷-(C=O)ₙ-R⁸, -NR⁷-SO₂-R⁸, -NR⁷-C(=O)-OR⁸, -NR⁹-C(=O)-NR⁷R⁸ or -NR⁹-SO₂-NR⁷R⁸,
in which
n represents the number 0 or 1,
p represents the number 0 or 2,
R⁷, R⁸ and R⁹ each independently of one another represent hydrogen, (C₁-C₆)-alkyl, (C₂-C₆)-alkenyl, (C₃-C₇)-cycloalkyl, (C₃-C₇)-cycloalkenyl, phenyl, 5- to 7-membered heterocyclyl or 5- or 6-membered heteroaryl,
in which R⁷, R⁸ and R⁹ for their part may be substituted by 1 to 3 substituents independently of one another selected from the group consisting of fluorine, chlorine, cyano, trifluoromethyl, (C₁-C₄)-alkyl, hydroxyl, trifluoromethoxy, (C₁-C₄)-alkoxy, oxo, amino, mono-(C₁-C₄)-alkylamino and di-(C₁-C₄)-alkylamino,
or
R⁷ and R⁸ together with the radical to which the two are attached form a 5- to 7-membered heterocycle,
or
R⁷ and R⁹ together with the radical to which the two are attached form a 5- to 7-membered heterocycle,
and also *N*-oxides, salts, solvates, salts of the *N*-oxides and solvates of the *N-*oxides and salts thereof.

3. Compound of the formula (I) according to Claim I or 2 in which
A represents N,
D represents CR⁴,
where
R⁴ represents hydrogen,
E represents CR⁵,
where
R⁵ represents hydrogen,
G represents CR⁶,
where
R⁶ represents hydrogen,
R¹ represents cyclohexyl or cycloheptyl,
R² represents a group of the formula 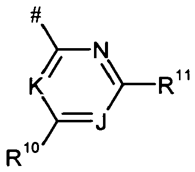 where
# represents the point of attachment to the heterobicycle,
K represents N,
J represents CR¹² or N,
in which
R¹² represents hydrogen, -R⁷, -NR⁷-(C=O)ₙ-R⁸, -NR⁷-C(=O)-OR⁸ or pyridyl,
in which
n represents the number 1,
R⁷ represents hydrogen, trifluoromethyl or (C₁-C₄)-alkyl,
in which (C₁-C₄)-alkyl for its part may be substituted by a substituent selected from the group consisting of fluorine, trifluoromethyl, hydroxyl and methoxy,
R⁸ represents (C₁-C₄)-alkyl or (C₃-C₇)-cycloalkyl,
in which (C₁-C₄)-alkyl and (C₃-C₇)-cycloalkyl for their part may be substituted by a substituent selected from the group consisting of fluorine, trifluoromethyl, (C₁-C₄)-alkyl, hydroxyl, methoxy and oxo,
or
R⁷ and R⁸ together with the radical to which the two are attached form a 5- to 7-membered heterocycle,
and also *N*-oxides, salts, solvates, salts of the *N*-oxides and solvates of the *N-*oxides and salts thereof.

4. Process for preparing compounds of the formula (I) as defined in any of Claims 1 to 3, **characterized in that** a compound of the formula (II) 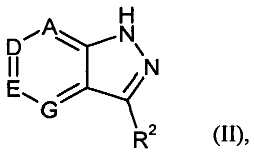 in which A, D, E, G and R² each have the meanings given in any of Claims 1 to 3 is reacted in an inert solvent in the presence of a suitable base with a compound of the formula (III) 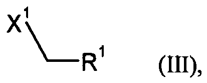 in which R¹ has the meaning given in any of Claims 1 to 3
and
X¹ represents a suitable leaving group, such as tosylate, mesylate or halogen, in particular bromine,
the resulting compound of the formula (I) is, where appropriate, modified within the scope, given above, of the meanings of the individual substituents and radicals using processes customary in the literature
and the resulting compounds of the formula (I) are, where appropriate, converted with the appropriate (i) solvents and/or (ii) bases or acids into their solvates, salts and/or solvates of the salts.

5. Compound of the formula (I) as defined in any of Claims 1 to 3 for the treatment and/or prophylaxis of diseases.

6. Compound of the formula (I) as defined in any of Claims I to 3 for use in a method for the treatment and/or prevention of heart failure, angina pectoris, hypertension, pulmonary hypertension, ischemias, vascular disorders, thromboembolic disorders and arteriosclerosis.

7. Use of a compound of the formula (I) as defined in any of Claims 1 to 3 for the preparation of a medicament for the treatment and/or prevention of heart failure, angina pectoris, hypertension, pulmonary hypertension, ischemias, vascular disorders, thromboembolic disorders and arteriosclerosis.

8. Medicament comprising a compound of the formula (I) as defined in any of Claims 1 to 3 in combination with an inert, non-toxic, pharmaceutically suitable excipient

9. Medicament comprising a compound of the formula (I) as defined in any of Claims 1 to 3 in combination with a further active ingredient selected from the group consisting of organic nitrates, NO donors, cGMP-PDE inhibitors, agents having antithrombotic activity, agents lowering blood pressure, and agents altering lipid metabolism.

10. Medicament according to Claim 8 or 9 for the treatment and/or prevention of heart failure, angina pectoris, hypertension, pulmonary hypertension, ischemias, vascular disorders, thromboembolic disorders and arteriosclerosis.

## Revendications

1. Composé de formule (I) 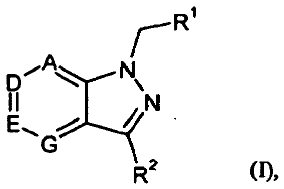 dans laquelle
A représente CR³ ou N,
R³ représentant hydrogène, halogène, cyano, alkyle en (C₁-C₄), trifluorométhyle, amino, alcoxy en (C₁-C₄) et trifluorométhoxy,
D représente CR⁴ ou N,
R⁴ représentant hydrogène, halogène, cyano, alkyle en (C₁-C₄), trifluorométhyle, amino, alcoxy en (C₁-C₄) et trifluorométhoxy,
E représente CR⁵ ou N,
R⁵ représentant hydrogène, halogène, cyano, alkyle en (C₁-C₄), trifluorométhyle, amino, alcoxy en (C₁-C₄) et trifluorométhoxy,
G représente CR⁶ ou N,
R⁶ représentant hydrogène, halogène, cyano, alkyle en (C₁-C₄), trifluorométhyle, amino, alcoxy en (C₁-C₄) et trifluorométhoxy,
à condition qu'au plus 2 des groupes A, D, E et G représentent N,
R¹ représente cycloalkyle en (C₃-C₈), cycloalkyle en (C₃-C₈) pouvant être substitué avec 1 ou 2 substituants choisis indépendamment l'un de l'autre dans le groupe fluor et alkyle en (C₁-C₄), R² représente pyrrolinyle, dihydropyrazolyle, imidazolinyle, dihydrooxazolyle, dihydroisoxazolyle, dihydro-1,2,4-triazolyle, dihydro-1,2,4-oxadiazolyle, dihydro-1,3,4-oxadiazolyle, dihydro-1,2,4-thiadiazolyle, dihydropyranyle, 1,4-dihydropyridyle, tétrahydropyrimidinyle, 1,3-oxazinyle, furyle, thiényle, thiazolyle, oxazolyle, isothiazolyle, isoxazolyle, pyrazolyle, imidazolyle, triazolyle, oxadiazolyle, thiadiazolyle, pyridyle, pyrimidinyle, pyridazinyle, pyrazinyle ou triazinyle, pyrrolinyle, dihydropyrazolyle, imidazolinyle, dihydrooxazolyle, dihydroisoxazolyle, dihydro-1,2,4-triazolyle, dihydro-1,2,4-oxadiazolyle, dihydro-1,3,4-oxadiazolyle, dihydro-1,2,4-thiadiazolyle, dihydropyranyle, 1,4-dihydropyridyle, tétrahydropyrimidinyle, 1,3-oxazinyle, furyle, thiényle, thiazolyle, oxazolyle, isothiazolyle, isoxazolyle, pyrazolyle, imidazolyle, triazolyle, oxadiazolyle, thiadiazolyle, pyridyle, pyrimidinyle, pyridazinyle, pyrazinyle et triazinyle pouvant être substitués avec 1 à 4 substituants choisis indépendamment les uns des autres dans le groupe constitué par halogène, azido, nitro, cyano, -R⁷, -C(=O)-R⁷, -C(=O)-OR⁷, -C(=O)-NR⁷R⁸, -O-(C=O)ₙ-R⁷, -O-C(=O)-OR⁷, -O-C(=O)-NR⁷R⁸, -S(O)ₚ-R⁷, -SO₂-OR⁷, -SO₂-NR⁷R⁸, -NR⁷-(C=O)ₙ-R⁸, -NR⁷-SO₂-R⁸, -NR⁷-C(=O)-OR⁸, -NR⁹-C(=O)-NR⁷R⁸ et -NR⁹-SO₂-NR⁷R⁸,
n représentant un nombre 0 ou 1,
p représentant un nombre 0, 1 ou 2,
R⁷, R⁸ et R⁹ représentant chacun indépendamment les uns des autres hydrogène, alkyle en (C₁-C₆), alcényle en (C₂-C₆), cycloalkyle en (C₃-C₈), cycloalcényle en (C₃-C₈), aryle en (C₆-C₁₀), hétérocyclyle de 4 à 8 éléments ou hétéroaryle de 5 à 10 éléments,
R⁷, R⁸ et R⁹ pouvant de leur côté être substitués avec 1 à 5 substituants choisis indépendamment les uns des autres dans le groupe halogène, azido, nitro, cyano, trifluorométhyle, alkyle en (C₁-C₆), alkylcarbonyle en (C₁-C₆), alkylcarbonyloxy en (C₁-C₆), hydroxycarbonyle, alcoxycarbonyle en (C₁-C₆), aminocarbonyle, mono-alkylaminocarbonyle en (C₁-C₆), di-alkylaminocarbonyle en (C₁-C₆), hydroxy, trifluorométhoxy, alcoxy en (C₁-C₆), oxo, mercapto, alkylthio en (C₁-C₆), amino, mono-alkylamino en (C₁-C₆), di-alkylamino en (C₁-C₆), formylamino, alkylcarbonylamino en (C₁-C₆), alcoxycarbonylamino en (C₁-C₆), cycloalkyle en (C₃-C₈), cycloalcényle en (C₃-C₈), ainsi qu'hétérocyclyle de 4 à 8 éléments,
ou
R⁷ et R⁸ formant ensemble avec le radical auquel ils sont tous les deux reliés un hétérocycle de 4 à 8 éléments,
ou
R⁷ et R⁹ formant ensemble avec le radical auquel ils sont tous les deux reliés un hétérocycle de 4 à 8 éléments,
ainsi que leurs N-oxydes, sels, solvates, sels des N-oxydes et solvates des N-oxydes et des sels.

2. Composé de formule (I) selon la revendication 1, dans lequel
A représente CR³ ou N,
R³ représentant hydrogène,
D représente CR⁴ ou N,
R⁴ représentant hydrogène,
E représente CR⁵ ou N,
R⁵ représentant hydrogène,
G représente CR⁶ ou N,
R⁶ représentant hydrogène,
à condition qu'au plus 2 des groupes A, D, E et G représentent N,
R¹ représente cyclopentyle, cyclohexyle ou cycloheptyle,
cyclopentyle, cyclohexyle ou cycloheptyle pouvant être substitué avec 1 ou 2 substituants choisis indépendamment l'un de l'autre dans le groupe fluor et méthyle,
R² représente un groupe de formule 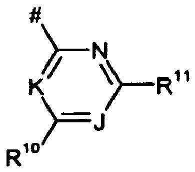 # représentant l'emplacement de liaison à l'hétérobicycle,
K représentant CH ou N,
J représentant CR¹², N ou N⁺-O⁻, R¹² représentant halogène, nitro, cyano, -R⁷, -C(=O)-R⁷, -C(=O)-OR⁷, -C(=O)-NR⁷R⁸, -O-(C=O)ₙ-R⁷, -O-C(=O)-OR⁷, -O-C(=O)-NR⁷R⁸, -S(O)ₚ-R⁷, -SO₂-OR⁷, -SO₂-NR⁷R, -NR⁷-(C=O)ₙ-R⁸, -NR⁷SO₂-R⁸, -NR⁷-C(=O)-OR⁸, -NR⁹-C(=O)-NR⁷R⁸ et -NR⁹-SO₂-NR⁷R⁸,
n représentant le nombre 0 ou 1,
p représentant le nombre 0 ou 2,
R⁷, R⁸ et R⁹ représentant chacun indépendamment les uns des autres hydrogène, alkyle en (C₁-C₆), alcényle en (C₂-C₆), cycloalkyle en (C₃-C₇), cycloalcényle en (C₃-C₇), phényle, hétérocyclyle de 5 à 7 éléments ou hétéroaryle de 5 ou 6 éléments,
R⁷, R⁸ et R⁹ pouvant de leur côté être substitués avec 1 à 3 substituants choisis indépendamment les uns des autres dans le groupe fluor, chlore, cyano, trifluorométhyle, alkyle en (C₁-C₄), hydroxy, trifluorométhoxy, alcoxy en (C₁-C₄), oxo, amino, mono-alkylamino en (C₁-C₄) et di-alkylamino en (C₁-C₄),
ou
R⁷ et R⁸ formant ensemble avec le radical auquel ils sont tous les deux reliés un hétérocycle de 5 à 7 éléments,
ou
R⁷ et R⁹ formant ensemble avec le radical auquel ils sont tous les deux reliés un hétérocycle de 5 à 7 éléments,
ainsi que leurs N-oxydes, sels, solvates, sels des N-oxydes et solvates des N-oxydes et des sels.

3. Composé de formule (I) selon la revendication 1 ou 2, dans lequel
A représente N,
D représente CR⁴,
R⁴ représentant hydrogène,
E représente CR⁵,
R⁵ représentant hydrogène,
G représente CR⁶,
R⁶ représentant hydrogène,
R¹ représente cyclohexyle ou cycloheptyle,
R² représente un groupe de formule 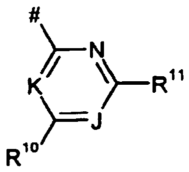 # représentant l'emplacement de liaison à l'hétérobicycle,
K représentant N,
J représentant CR¹² ou N,
R¹² représentant hydrogène, -R⁷, -NR⁷-(C=O)ₙ-
R⁸, -NR⁷-C(=O)-OR⁸ ou pyridyle,
n représentant le nombre 1,
R⁷ représentant hydrogène, trifluorométhyle ou alkyle en (C₁-C₄),
alkyle en (C₁-C₄) pouvant de son côté être substitué avec un substituant choisi dans le groupe fluor, trifluorométhyle, hydroxy et méthoxy,
R⁸ représentant alkyle en (C₁-C₄) ou cycloalkyle en (C₃-C₇),
alkyle en (C₁-C₄) ou cycloalkyle en (C₃-C₇) pouvant de leur côté être substitués avec un substituant choisi dans le groupe fluor, trifluorométhyle, alkyle en (C₁-C₄), hydroxy, méthoxy et oxo,
ou
R⁷ et R⁸ formant ensemble avec le radical auquel ils sont tous les deux reliés un hétérocycle de 5 à 7 éléments,
ainsi que leurs N-oxydes, sels, solvates, sels des N-oxydes et solvates des N-oxydes et des sels.

4. Procédé de fabrication de composés de formule (I), tels que définis dans les revendications 1 à 3, **caractérisé en ce qu'**un composé de formule (II) 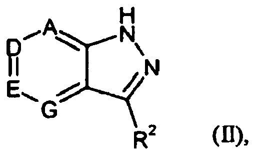 dans laquelle A, D, E, G et R² ont chacun les significations indiquées dans les revendications 1 à 3,
est mis en réaction dans un solvant inerte en présence d'une base appropriée avec un composé de formule (III) 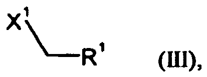 dans laquelle R¹ a la signification indiquée dans les revendications 1 à 3,
et
X¹ représente un groupe partant approprié, tel que tosylate, mésylate ou halogène, notamment brome,
le composé de formule (I) résultant est éventuellement davantage modifié par des procédés usuels dans la littérature dans le cadre des significations indiquées précédemment des substituants et radicaux individuels,
et les composés de formule (I) résultants sont éventuellement transformés en leurs solvates, sels et/ou solvates des sels avec les (i) solvants et/ou (ii) bases ou acides correspondants.

5. Composé de formule (I), tel que défini dans l'une quelconque des revendications 1 à 3, pour le traitement et/ou la prophylaxie de maladies.

6. Composé de formule (I), tel que défini dans l'une quelconque des revendications 1 à 3, destiné à être utilisé dans un procédé pour le traitement et/ou la prévention de l'insuffisance cardiaque, de l'angine de poitrine, de l'hypertension, de l'hypertension pulmonaire, des ischémies, des angiopathies, des maladies thromboemboliques et de l'artériosclérose.

7. Utilisation d'un composé de formule (I), tel que défini dans l'une quelconque des revendications 1 à 3, pour la fabrication d'un médicament pour le traitement et/ou la prévention de l'insuffisance cardiaque, de l'angine de poitrine, de l'hypertension, de l'hypertension pulmonaire, des ischémies, des angiopathies, des maladies thromboemboliques et de l'artériosclérose.

8. Médicament contenant un composé de formule (I), tel que défini dans l'une quelconque des revendications 1 à 3, en combinaison avec un adjuvant inerte, non toxique, pharmaceutiquement approprié.

9. Médicament contenant un composé de formule (I), tel que défini dans l'une quelconque des revendications 1 à 3, en combinaison avec un agent actif supplémentaire choisi dans le groupe constitué par les nitrates organiques, les donneurs de NO, les inhibiteurs de cGMP-PDE, les agents à action antithrombotique, les agents abaissant la tension artérielle, ainsi que les agents modifiant le métabolisme des lipides.

10. Médicament selon la revendication 8 ou 9 pour le traitement et/ou la prévention de l'insuffisance cardiaque, de l'angine de poitrine, de l'hypertension, de l'hypertension pulmonaire, des ischémies, des angiopathies, des maladies thromboemboliques et de l'artériosclérose.
